# EUROPÄISCHE PATENTSCHRIFT

(11) **EP 2 478 932 B1**
(45) Veröffentlichungstag und Bekanntmachung des Hinweises auf die Patenterteilung: **04.06.2014**
(21) Anmeldenummer: 11192602.8
(22) Anmeldetag: 08.12.2011
(51) Int. Cl.: A61N 1/08, A61N 1/372

(54) **Implantierbares Gerät**
Implantable device
Appareil implantable

(30) Priorität: 20.12.2010 US 201061424685 P
(43) Veröffentlichungstag der Anmeldung: 25.07.2012
(73) Patentinhaber: BIOTRONIK SE & Co. KG, 12359 Berlin (DE)
(72) Erfinder: Dörr, Thomas, 12437 Berlin (DE); Weiss, Ingo, 12435 Berlin (DE)
(74) Vertreter: Lindner-Vogt, Karin L.

(56) Entgegenhaltungen:
- GB-A- 1 410 743
- US-A- 4 387 717
- US-A- 4 532 934
- US-A1- 2003 204 207

## Beschreibung

Die Erfindung betrifft ein permanent oder temporär implantierbares Gerät mit einem langgestreckten elektrischen Leiter.

Solche Geräte, beispielsweise Elektrodenleitungen für die Elektrostimulation, haben den Nachteil, dass sich ihr elektrischer Leiter in einem Kernspintomografen erwärmen kann, weil die im Kernspintomografen herrschenden wechselnden Magnetfelder in dem elektrischen Leiter nicht unbeachtliche elektrische Ströme induzieren. Auch können solche induzierten Ströme über Elektrodenpole der Elektrodenleitung an umliegendes Gewebe abgegeben werden und so beispielsweise zu unerwünschten Gewebeerwärmungen führen. Deshalb können Herzschrittmacherpatienten heutzutage in der Regel nicht oder nur eingeschränkt in einem Kernspintomografen untersucht werden.

An implantierbaren Herzschrittmachern oder Defibrillatoren (im Folgenden auch gemeinsam als Herzstimulatoren oder IPG (implantable pulse generator; implantierbarer Impulsgenerator) bezeichnet) sind nämlich typischerweise wenigstens eine Stimulationselektrodenleitung angeschlossen, die an ihrem proximalen, zum Anschluss an den Herzschrittmacher oder Defibrillator vorgesehenen Ende einen standardisierten elektrischen Anschluss aufweist und an ihrem distalen, zur Platzierung im Herzen vorgesehenen Ende einen oder mehrere Elektrodenpole aufweist. Ein solcher Elektrodenpol dient zur Abgabe elektrischer Impulse an das Gewebe (Myokard) des Herzens oder zum Abfühlen elektrischer Felder, um im Rahmen des sogenannten Sensings eine Aktivität eines Herzens abfühlen zu können. Zu diesen Zwecken bilden Elektrodenpole typischerweise elektrisch leitende Oberflächenabschnitte einer Elektrodenleitung. Elektrodenpole sind typischerweise als Ringelektrode in Form eines Rings um die Elektrodenleitung oder in Form einer Spitzen- oder Tippelektrode am distalen Ende der Elektrodenleitung vorgesehen. Die Elektrodenpole sind über eine oder mehrere elektrische Leiter mit Kontakten des elektrischen Anschlusses der Elektrodenleitung an deren proximalem Ende elektrisch leitend verbunden. Somit verlaufen zwischen den Kontakten des elektrischen Anschlusses die Elektrodenleitungen an deren proximalen Ende und den Elektrodenpolen am distalen Ende der Elektrodenleitung ein oder mehrere elektrische Leiter, die einen oder mehrere der Elektrodenpole mit einem oder mehreren der Kontakte elektrisch verbinden. Diese elektrischen Leiter können einerseits zur Übertragung von Stimulationsimpulsen zu den Elektrodenpolen und andererseits zur Übertragung mittels der Elektrodenpole aufgenommener elektrischer Signale zum proximalen Ende der Elektrodenleitung genutzt werden und werden im Verlauf der weiteren Beschreibung auch jeweils als Funktionsleitung bezeichnet. Solche Funktionsleitungen sind für die Funktionen der jeweiligen Elektrodenleitung erforderliche elektrische Leiter und als solche der Gefahr ausgesetzt, dass in ihnen durch äußere Wechselmagnetfelder elektrische Ströme induziert werden, die beispielsweise zu einer unerwünschten Erwärmung der Funktionsleitungen oder der mit ihr verbundenen Elektrodenpole führen können oder die zur Abgabe entsprechender Ströme über die Elektrodenpole an umgebendes Gewebe und damit zu einer Erwärmung des umgebenden Gewebes führen können.

Ein Gerät gemäß Präambel von Anspruch 1 ist schon aus GB 1410743 bekannt.

Der Erfindung liegt die Aufgabe zugrunde, ein Gerät zu schaffen, welches das zuvor beschriebene Problem löst.

Erfindungsgemäß wird diese Aufgabe durch ein temporär oder permanent implantierbares medizinisches Gerät mit wenigstens einem langgestreckten elektrischen Funktionsleiter für die Übertragung von Therapiesignalen oder Diagnosesignalen oder beidem, und einem mit dem Funktionsleiter verbundenen Elektrodenpol, über den elektrischer Strom an im Benutzungsfall an umgebendes Körpergewebe abgegeben oder mit dem elektrische Potentiale in im Benutzungsfall umgebenden Gewebe abgefühlt werden können oder beides, gelöst, bei dem eine Wahrnehmungseinrichtung vorgesehen ist, die mit einem felderzeugenden sowie einem potentialabfühlenden Elektrodenpol verbunden und ausgebildet ist, im Falle einer Stromabgabe über den felderzeugenden Elektrodenpol generierter elektrischer Potentiale über den potentialabfühlenden Elektrodenpol mit Bezug auf ein Bezugspotential zu erfassen und ein ein erfasstes elektrisches Potential repräsentierendes Ausgangssignal zu erzeugen,
sowie eine Steuereinrichtung, die mit der Wahrnehmungseinrichtung verbunden und ausgebildet ist, ein von der Wahrnehmungseinrichtung erzeugte Ausgangssignal auszuwerten und das medizinische Gerät in Abhängigkeit des von der Wahrnehmungseinrichtung erfassten Potentials zu steuern.

Die Erfindung schließt ein implantiertes System mit angeschlossener Elektrodenleitung mit mindestens 2 Elektrodenpolen oder ein langgestrecktes elektronisches Implantat mit Elektrodenpolen ein, deren elektrisches Feld des einen Elektrodenpols als felderzeugenden Elektrodenpol infolge induzierter Ströme z.B. durch Wechselwirkung im Magnetresonanztomografen (MRT) das umliegende Gewebe erwärmen kann von einer weiteren Elektrodenpolanordnung als wahrnehmende Elektrodenpole wahrgenommen wird und dabei ein oder mehrere Signal erzeugt, das von einer mit den Elektrodenpolen verbunden Einrichtung ausgewertet wird und zur Ansteuerung des Implantates im Hinblick auf eine sicherheitstechnische Maßnahme genutzt wird.

Da das zu erfassende Potential ein Potential infolge hochfrequenter, äußerer Störfelder ist, ist das Potential selbst ein Signal mit hochfrequentem Signalverlauf.

Der felderzeugende Elektrodenpol kann gleichzeitig einer der wahrnehmenden Elektrodenpole sein, zu denen wenigstens ein Referenzpol als weiterer Elektrodenpol zählt, der das bezugspotential liefert.

Vorzugsweise werden als nicht felderzeugende, wahrnehmende Elektrodenpole gewählt, die ohnehin als therapeutische/diagnostische Pole auf der Elektrode angebracht sind, so dass sich zusätzliche Elektrodenpole erübrigen.

Als das Bezugspotential liefernder Referenzpol für das Signal des wahrnehmenden Elektrodenpols dient vorzugsweise der implantatsseitige Eingang, der zur Verbindung mit dem der felderzeugende Elektrodenpol vorgesehen ist.

Alternativ kann auch als das Bezugspotential liefernder Referenzpol für das Signal des wahrnehmenden Elektrodenpols ein implantatsseitiger Eingang vorgesehen sein, der zur Verbindung mit einem dritten Elektrodenpol vorgesehen ist.

Alternativ kann auch als das Bezugspotential liefernder Referenzpol für das Signal des wahrnehmenden Elektrodenpols ein von einem Implantatsgehäuse gebildeter Elektrodenpol vorgesehen sein.

Grundsätzlich kann das medizinische Gerät auch dazu ausgebildet sein, ein Kombination unterschiedlicher Signale, die z.B. von verschiedenen Wahrnehmenden Elektrodenpolen stammen gegenüber unterschiedlichen Bezugspotentialen erfasst werden, zu verarbeiten. Dazu kann die Wahrnehmungseinrichtung einen Mischer enthalten um Signale zusammenzuführen. Eine Auswerteeinheit oder Auswerteeinrichtung der Wahrnehmungseinheit kann alternativ auch dazu ausgebildet sein, die verschiedenen Signale parallel, also nicht gemischt, zu verarbeiten. In beiden Fällen ergibt sich eine höhere Störsicherheit.

Die Auswerteeinrichtung kann außerdem dazu ausgebildet sein, nur Signale in bestimmten Frequenzbereichen auszuwerten, z.B. Signale > 1 MHz, oder Bänder mit Bandbreiten von <5MHz um typische MR HF Frequenzen wie z.B. 42, 64, 128 MHz. Hierzu kann die Wahrnehmungseinrichtung ein entsprechendes Filter aufweisen.

Weiterhin kann die Auswerteeinrichtung dazu ausgebildet sein, nur Signale oberhalb einer bestimmten Schwelle auszuwerten, z.B. im Frequenzbereich um 64 MHz > 1V.

Als wahrnehmender Elektrodenpol ist vorzugsweise ein Elektrodenpol mit geringem Erwärmungspotential vorgesehen, d.h. ein Elektrodenpol, der geringe Störung erwarten lässt. Die Auswerteeinrichtung kann dazu ausgebildet sein, den wahrnehmende Elektrodenpol unter den zur Verfügung stehenden Elektrodenpolen auszuwählen. beispielsweise kann als felderzeugender Elektrodenpol eine Tip-Elektrode einer koaxial aufgebauten Standardelektrode gewählt sein. Als wahrnehmender Elektrodenpol wir entsprechend eine Ring-Elektrode gewählt.

Gemäß einer bevorzugten Ausführungsvariante ist die Wahl der Elektrodenpole (felderzeugend, wahrnehmend) extern programmierbar.

Die Auswerteeinrichtung kann dazu ausgebildet sein, den kumulativen Effekt beispielsweise durch Integralbildung, Mittelwertbildung oder Bestimmung des Effektivwertes des das erfasste Potential repräsentierenden Signals zu bestimmen.

Alternativ kann die Auswerteeinrichtung dazu ausgebildet sein, den maximalen Effekt des i.d.R. gepulsten, das erfasste Potential repräsentierenden Signals zu bestimmen und hierzu beispielsweise als Maximalwert-Detektor ausgebildet sein.

Die Steuereinrichtung ist vorzugsweise dazu ausgebildet, auf ein entsprechendes Ausgangssignal der Auswerteeinrichtung hin das medizinische Gerät und/oder implantatsseitige Elektrodenbeschaltung in einen für die jeweilige Betriebsumgebung, insbesondere im Falle extremer elektromagnetischer Feldeinwirkung, sicheren Modus zu schalten, wenn des Ausgangssignal der Auswerteeinrichtung eine solche Betriebsumgebung anzeigt. Vorzugsweise ist die Steuereinrichtung dazu ausgebildet, dieses umschalten zeitlich begrenzt, z.B. für eine bestimmte, programmierbare Zeit oder eine Latenzzeit nach letzter registrierter Störung vorzunehmen.

Gemäß einer weiteren bevorzugten Ausführungsvariante kann das medizinische Gerät eine Telemetrieeinrichtung aufweisen, die wenigstens mittelbar mit der Auswerteeinrichtung verbunden ist. Die Auswerteeinrichtung ist in diesem Fall vorzugsweise dazu ausgebildet, ein Auftreten einer überschwelligen Elektrodenerwärmung oder eine hierauf hindeutende elektrische Signalamplitude über die Telemetrieeinheit nach außen zu kommunizieren. Dies kann in Echtzeit erfolgen oder nach der Untersuchung zur Information des Arztes über beispielsweise ein Home Monitoring System.

Vorzugsweise weist das medizinische Gerät- insbesondere wenn es die Form einer Elektrodenleitung hat - distal in unmittelbarer Nähe der wahrnehmenden Elektrodenpole einen Wandler auf, der die an den wahrnehmenden Elektrodenpolen erfasste Signalenergie zur effizienteren (ungestörten) Übertragung zur Auswerteeinheit umwandelt.

Der Wandler kann ein Transformator sein, der das erfasste Potentiale repräsentierende Signal hochtransformiert um es über hochohmige Leitungen und damit RF störungsfest zur Auswerteeinheit zu leiten.

Alternativ kann der Wandler ist ein piezoelektrischer Wandler oder allgemein ein elektroakustischer Wandler sein, der erfasste Potentiale in mechanische Signale wandelt, so dass diese akustisch, also mechanisch und damit RF störungsfest zur Auswerteeinheit geleitet werden können.

Gemäß einer weiteren Alternative ist der Wandler ein Gleichrichter oder Demodulator, der die erfassten Potentiale in ein Nutzsignal in Form eines Gleichsignals wandelt. Eventuelle Störungen (RF) können dann vor der Auswerteeinheit leicht herausgefiltert werden.

Eine weitere Alternative stellt ein Wandler in Form eines elektrooptischen Wandlers dar, z.B. einer Leuchtdiode (LED), der erfasste Potentiale in ein Nutzsignal in Form eines Lichtsignals wandelt, dass dann ungestört zur Auswerteeinrichtung geleitet werden kann. Wenn das Nutzsignal ein mechanisches, insbesondere akustisches, oder ein optisches Nutzsignal ist, kann in der Nähe der Auswerteeinheit, z.B. am proximalen Ende einer Elektrodenleitung einer zweiter Wandler vorgesehen, der das jeweilige Nutzsignal wieder in ein elektrisches Signal wandelt. Da dies vorteilhafterweise erst kurz vor der Auswerteeinheit geschieht, ist die Störempfindlichkeit entsprechend vermindert.

Die Erfindung soll nun anhand von Ausführungsbeispielen mit Bezug auf die Figuren näher erläutert werden. Die Figuren zeigen Folgendes:
- FIG. 1: zeigt als implantierbare medizinische Geräte einen implantierbaren Herzstimulator 10 und eine daran angeschlossene implantierbare Elektrodenleitung 20.
- FIG. 2: zeigt einen typischen Temperaturverlauf an der Elektrodenspitze.
- FIG. 3: zeigt am Beispiel, wie ein felderzeugender Elektrodenpol (Tip-Elektrode) (eines Leiters mit starker Störeinkopplung) und wahrnehmende Elektrodenpole in Form von Ringelektroden zusammenwirken. E*dl erzeugt eine Spannung die ein Maß für die Feldstärke E und damit für die Erwärmung des Tips ist.
- FIG. 4A und 4B: zeigen Felder an der Elektrodenspitze; der felderzeugende Elektrodenpol (hier die Tip-Elektrode) ist hier gleichzeitig einer der wahrnehmenden Pole
- FIG. 5A und 5B: zeigen zwei Varianten eines implantierbaren Impulsgenerators mit jeweils einer Einrichtung zur Auswertung eines elektrischen Signals der Elektrode, das zur Erwärmung eines Elektrodenpols korreliert.

Der implantierbare Herzstimulator 10 kann ein Herzschrittmacher oder ein Kardioverter/Defibrillator (ICD) sein. Im dargestellten Ausführungsbeispiel ist der Herzstimulator 10 ein ventrikulärer Herzschrittmacher und Defibrillator. Andere bekannte Herzstimulatoren sind Zweikammerherzschrittmacher zur Stimulation des rechten Atriums und des rechten Ventrikels oder biventrikuläre Herzschrittmacher, die zusätzlich zum rechten Ventrikel auch den linken Ventrikel stimulieren können.

Derartige Stimulatoren besitzen typischerweise ein Gehäuse 12, das im Regelfall aus Metall besteht und somit elektrisch leitend ist und als großflächiger Elektrodenpol dienen kann. An der Außenseite des Gehäuses 12 ist typischerweise ein Anschlussgehäuse 14 befestigt, das auch als Header bezeichnet wird. Ein derartiger Header weist typischerweise Kontaktbuchsen zur Aufnahme von Steckkontakten auf. Die Kontaktbuchsen besitzen elektrische Kontakte 16, die über entsprechende Leiter mit einer in dem Gehäuse 12 des Herzstimulators 10 angeordneten Elektronik verbunden sind.

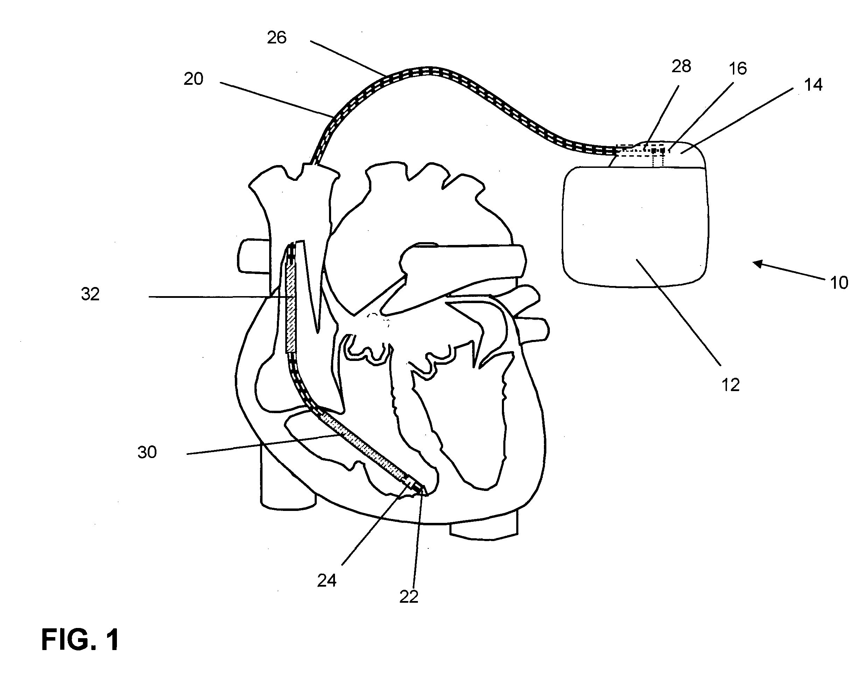

Die Elektrodenleitung 20 stellt im Sinne dieser Erfindung ebenfalls ein implantierbares medizinisches Gerät dar. Am distalen Ende der Elektrodenleitung 20 sind in an sich bekannter Manier Elektrodenpole in Form einer Spitzen- oder Tipelektrode 22 und einer in deren Nähe angeordneten Ringelektrode 24 angeordnet. Die Elektrodenpole 22 und 24 sind so ausgebildet, dass sie je nach Funktion eines Herzstimulators, an den die Elektrodenleitung 20 angeschlossen ist, zum Abfühlen elektrischer Potenziale des Herzgewebes (Myokards) dienen oder zur Abgabe elektrischer Signale, beispielsweise zur Abgabe von Stimulationsimpulsen an das sie umgebende Herzgewebe, ausgebildet sind. FIG. 1 zeigt, wie sich die Elektrodenpole, also die Tipelektrode 22 und die Ringelektrode 24, im Anwendungsfall die Elektrodenleitung 20, im Apex eines rechten Ventrikels eines Herzens befinden.

Sowohl die Tipelektrode 22 als auch die Ringelektrode 24 sind über jeweils wenigstens einen elektrischen Leiter 26 mit einem Steckkontakt 28 am proximalen Ende der Elektrodenleitung 20 elektrisch verbunden. Der Steckkontakt 28 besitzt elektrische Kontakte, die mit den elektrischen Kontakten 16 der Kontaktbuchse im Anschlussgehäuse 14 des implantierbaren Herzstimulators korrespondieren. Die elektrischen Leiter 26 in der Elektrodenleitung 20 können als annähernd gestreckte Seilzuleiter oder als helixförmig gewendelte Leiter ausgebildet sein. Solche Leiter, die funktionale Elektrodenpole mit elektrischen Kontakten des Steckkontaktes am proximalen Ende der Elektrodenleitung 20 elektrisch leitend verbinden werden im Rahmen dieses Textes als Funktionsleiter bezeichnet, da sie beispielsweise der Therapie dienende elektrische Signale von Steckkontakt zum jeweiligen Elektrodenpol übertragen oder abgefühlte elektrische Potentiale repräsentierende Signale vom jeweiligen Elektrodenpol zum Steckkontakt führen und somit der elementaren Funktion des medizinischen Gerätes dienen.

Die elektrischen Leiter 26, die die Elektrodenpole 22 bzw. 24 mit den elektrischen Kontakten des Steckers 28 der Elektrodenleitung 20 verbinden, sind über den größten Teil ihrer Länge von einer isolierenden Hülle umgeben, so dass ein elektrischer Kontakt zum Gewebe des Herzens gezielt über die Elektrodenpole zustande kommt.

Neben den Elektrodenpolen 22 und 24, die typischerweise der (in diesem Fall ventrikulären) Stimulation des Herzgewebes dienen, weist die Elektrodenleitung 20 auch noch zwei großflächigere Elektrodenpole 30 und 32 auf, die als Defibrillationselektroden dienen und von wenigstens einem blank liegendem helixartig gewendelten Draht gebildet sind.

Es sei darauf hingewiesen, dass die Erfindung im Rahmen dieses Ausführungsbeispiels anhand eines rechtsventrikulären Herzschrittmachers und Defibrillators erläutert wird. Als medizinisches Gerät im Sinne der Erfindung kann grundsätzlich aber beispielsweise auch eine Ablationselektrodenleitung dienen, die im Anwendungsfall ebenfalls bis ins Herz eines Patienten hineinragt und die von einem außerhalb des Patienten angeordneten Gerät gesteuert wird und hierzu mit diesem verbunden ist.

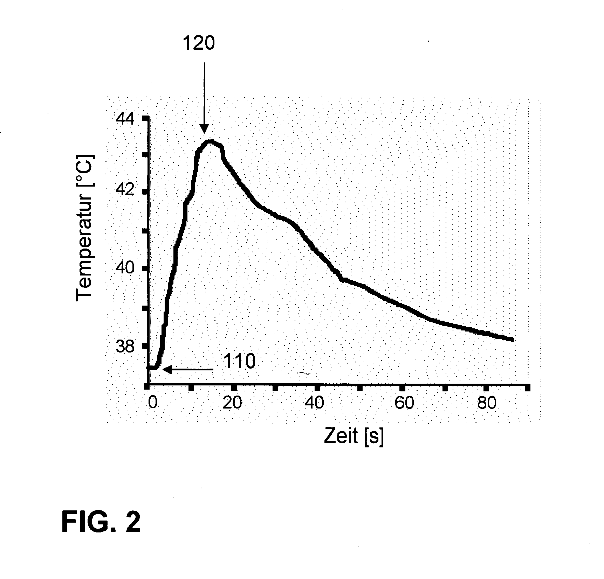

In Figur 2 ist ein typischer Temperaturverlauf 100 einer konventionellen Schrittmacher/ICD-Elektrode im Magnetresonanztomographen (MRT) dargestellt. Mit dem Einschalten des hochfrequenten Wechselfeldes im Tomographen zum Zeitpunkt 110 steigt die Temperatur rasch an, wobei die Steilheit des Anstieges und die maximal erreichbare Temperatur stark von der Elektrodenlage bezogen auf die hochfrequenten Wechselfelder des MRT abhängig ist. Wird das hochfrequente Wechselfeld abgeschaltet (zum Zeitpunkt 120), dann kühlt sich die Elektrodenspitze aufgrund ihrer vergleichsweise geringen Wärmekapazität verhältnismäßig schnell wieder ab.

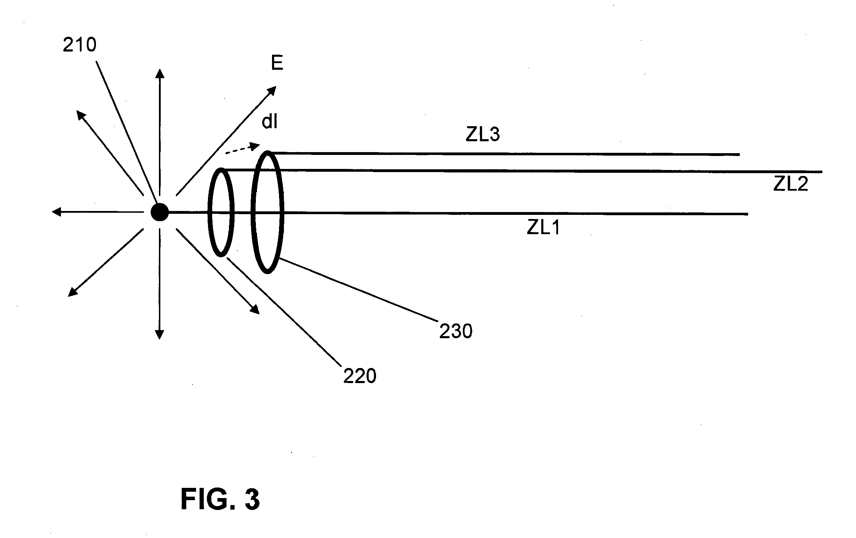

Figur 3 zeigt schematisch einen Elektrodenpol in Form einer Tipelektrode 210 sowie zwei Elektrodenpole in Form von Ringelektroden 220 und 230, die jeweils an einen Funktionsleiter (eine Zuleitung) ZL1, ZL2 beziehungsweise ZL3 angeschlossen sind. Die schematisch dargestellten Elektrodenpole befinden sich typischerweise am distalen Ende einer Elektrodenleitung, die darüber hinaus noch weitere Bestandteile, wie beispielsweise eine isolierende Hülle aufweist, die in der schematischen Darstellung jedoch nicht abgebildet sind. Wie bekannt, können die Funktionsleiter ZL1, ZL2 oder ZL3 verschiedene Formen annehmen und beispielsweise als Seilzuleiter oder auch als helixartig gewendelte Zuleiter ausgebildet sein. Figur 3 zeigt, wie ein felderzeugender Elektrodenpol, in diesem Falle die Tipelektrode 210, und wahrnehmende Elektrodenpole in Form der Ringelektroden 220 und 230 zusammenwirken, wenn die Funktionsleiter ZL1, ZL2, ZL3 einer starken Störeinkopplung, beispielsweise durch von außen einwirkende hochfrequente Wechselfelder in einem Magnetresonanztomographen (MRT), ausgesetzt sind. Das elektrische Feld um die Tipelektrode 210 ist durch Pfeile und dem Buchstaben E gekennzeichnet. Das Produkt aus E und der Strecke dl (E*dl) ergibt die Spannung, die aufgrund des von der Tipelektrode 210 ausgehenden Feldes mit der Feldstärke E zwischen den Ringelektroden 220 und 230 anliegt. Aus der Potentialdifferenz zwischen den Ringelektroden 220 und 230 kann so ein Maß für die Feldstärke des elektrischen Feldes E um die Tipelektrode 210 abgeleitet werden.

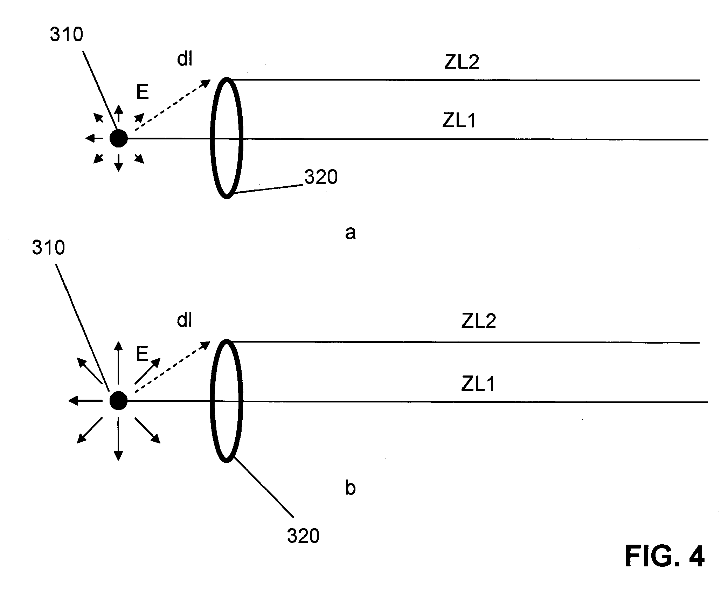

In Figuren 4A und 4B sind die Tip- und Ring-Elektroden 310 bzw. 320 und deren Zuleitungen (Funktionsleiter) ZL1 und ZL2 ähnlich schematisch dargestellt, wie in Figur 3.

Figuren 4A und 4B illustrieren, dass die am Elektrodenpol austretende Stromstärke je nach Stärke der einwirkenden elektromagnetischen Felder unterschiedlich groß ist. Damit korrelierend ändert sich auch das elektrische Feld E in seiner Stärke sowie die erzeugte Erwärmung. Für koaxiale Elektrodenleitungen (überwiegende Konstruktion in aktueller klinischer Praxis) ist beispielsweise die Erwärmung (und damit das E-Feld) der Ring-Elektrode(n) meist deutlich kleiner als das der Tip-Elektrode. Damit kann es näherungsweise vernachlässigt werden. Die Ring-Elektrode(n) 320 befindet sich aber im Feld der Tip-Elektrode 310 und liegt damit feldstärkeabhängig auf einem anderen Potential. Aus Integral ∫E(1)dl errechnet sich die entsprechende Spannung. Dieses Signal erreicht über die Funktionsleitung ZL2 (die Zuleitung für die Ring-Elektrode 320) den IPG und kann dort ausgewertet werden. Die Tatsache dass die Ringelektrode(n) im Feld der Tip-Elektrode auf einem bestimmten Potential liegt lässt sich im Bezug zu anderen Bezugs-potentialen feststellen (so z.B. der Tip selbst, ein anderer Ring oder das IPG Gehäuse).

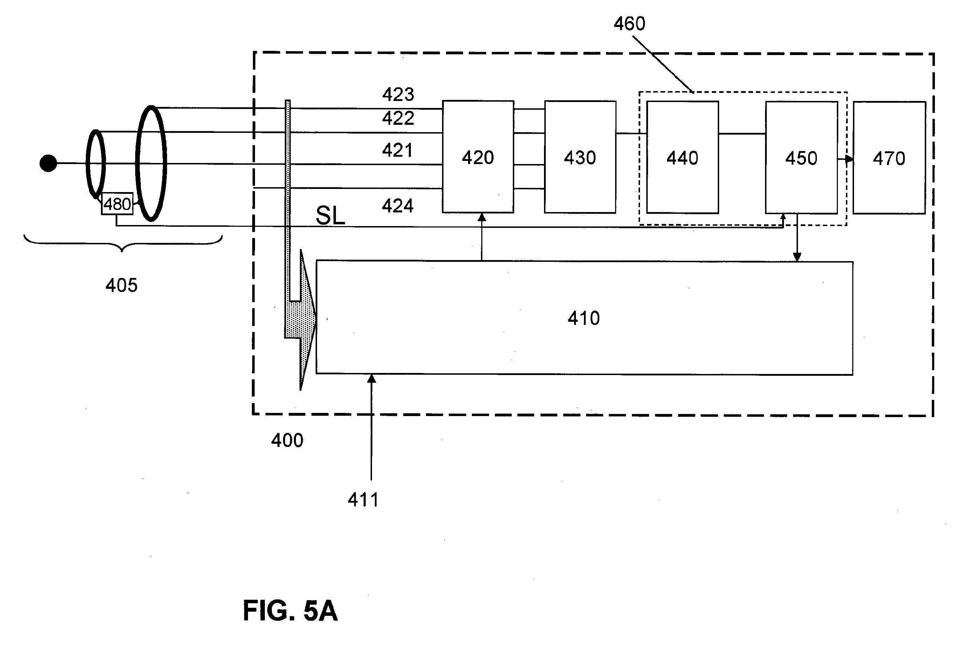

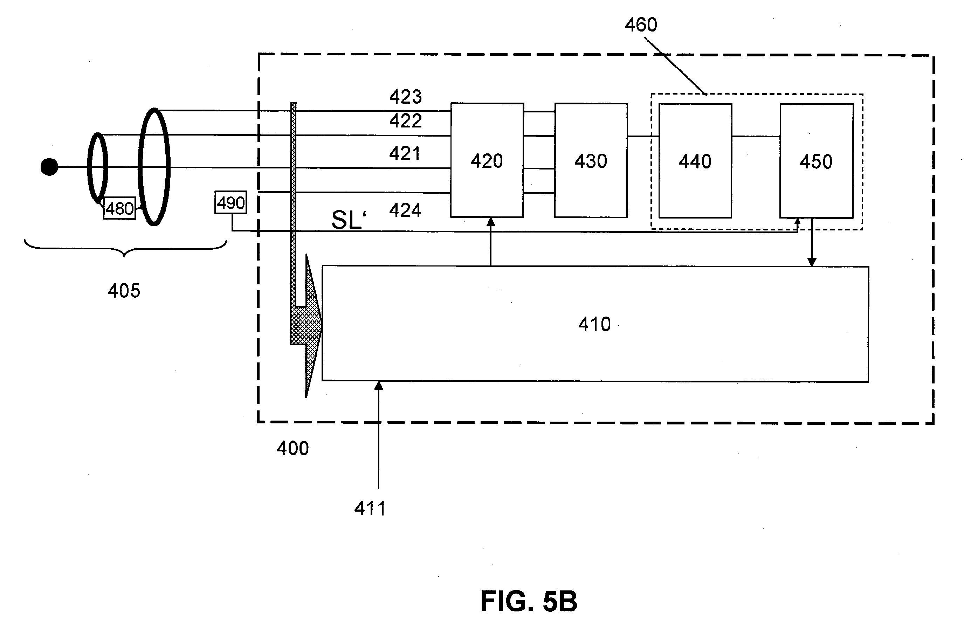

Figuren 5A und 5B zeigen jeweils ein Blockschaltbild des Herzstimulators 10 der hier als IPG (implantable pulse generator) bezeichnet ist. Der IPG besitz eine Wahrnehmungseinrichtung zur Auswertung eines elektrischen Signals der Elektrode das zur Erwärmung eines Elektrodenpols korreliert.

In Figuren 5A und 5B ist das IPG Gehäuse 400 des Herzstimulators 10 schematisch angedeutet. In dem IPG Gehäuse 400 sind blockartig die für das vorliegende Ausführungsbeispiel relevanten Komponenten dargestellt. Dass neben den dargestellten Komponenten noch weitere Komponenten eines typischen Herzstimulators vorhanden sind ist damit nicht ausgeschlossen. An das IPG Gehäuse 400 ist eine Elektrodenleitung 405 angeschlossen. Die Elektrodenleitung 405 ist ebenfalls schematisch im Sinne der Abbildung in Figur 3 dargestellt und besitzt im dargestellten Ausführungsbeispiel einen Elektrodenpol in Form einer Tipelektrode und zwei Elektrodenpole in Form von Ringelektroden, die jeweils über eine eigene Zuleitung (Funktionsleiter) 421, 422 beziehungsweise 423 mit einem optionalen Filter 420 verbunden sind. Dem optionalen Filter 420 ist ein Mischer 430 nachgeschaltet und dem Mischer 430 ein Schwellwertdetektor 440. Das Ausgangssignal des Schwellwertdetektors 440 wird einer Auswerteeinheit 450 zugeführt, die ihrerseits wieder auf die übrige Elektronik des Herzschrittmachers - allgemein als IPG Elektronik 410 bezeichnet - einwirkt. Mit einem Pfeil 411 ist angedeutet, dass die IPG Elektronik 410 von außen programmierbar ist.

Die Funktionsleiter (Zuleitungen) 421-423 der Elektrodenleitung 405 erreichen das Innere des IPG und gehen dort ein. Der optionale Filter 420 bewirkt, dass nur Signale in bestimmten Frequenzbereichen weiterverarbeitet werden, z.B. größer 1 MHz, oder Bänder mit Bandbreiten von weniger als 10MHz um typische Magnetresonanz HF Frequenzen wie z.B. 42, 64, 128 MHz.

Das IPG Gehäuse 400 kann ebenfalls als Elektrodenpol, z.B. als Referenzelektrode, dienen und ist zu diesem Zweck über eine Leitung 424 mit dem Filter 420 verbunden.

Diese Signale werden in dem Mischer 430 gewichtet summiert und dieses Ergebnis dem Schwellwertdetektor 440 zugefügt. Bei Vorliegen der Schwellwertbedingungen generiert der Schwellwertdetektor 440 ein entsprechendes Detektorausgangsignal. Dieses Detektorausgangssignal wird in der Auswerteeinheit 450 weiterverarbeitet und ein Steuersignal an die IPG Elektronik 410 gegeben. Die IPG Elektronik 410 bestimmt welche Funktionsleiter als Eingangsleitungen in das Filter eingeschaltet werten. Dies kann wiederum von extern programmiert werden (siehe Pfeil 411).

Bei den in Figuren 5A und 5B abgebildeten Ausführungsbeispielen bilden der Schwellwertdetektor 440 und die Auswerteeinheit 450 zusammengenommen eine Auswerteeinrichtung 460, die auf eine Schwellwertüberschreitung anspricht.

Die Auswerteeinrichtung 460 kann auch so ausgebildet sein, dass sie durch Integralbildung, Mittelwertbildung oder Bestimmung des Effektivwertes des Signals dessen kumulativen Effekt bestimmt und auswertet und ein Ausgangssignal in Abhängigkeit der Größe des kumulativen Effektes ausgibt.

Der Schwellwertdetektor 440 kann so ausgebildet sein, dass er auf den maximalen Wert seines Eingangssignals (das Ausgangssignal des Mischers 430) anspricht und in diesem Sinne als Maxwert Detektor fungiert.

Die IPG Elektronik 410 als Teil der Steuereinrichtung des Herzstimulators 10 kann so ausgebildet sein, dass sie den Herzstimulator 10 in Abhängigkeit des Ausgangssignals der Auswerteeinheit 450 beziehungsweise der Auswerteeinrichtung 460 immer dann in einen bei starker elektromagnetischer Feldeinwirkung sicheren Betriebsmodus schaltet und dies beispielsweise für eine bestimmte, vorprogrammierte Zeit tut.

Die Auswerteeinrichtung 460 kann auch mit einer Telemetrieeinheit 470 verbunden sein, um das Ausgangssignal der Auswerteeinrichtung auf ein externes Gerät übertragen zu können und so als Information für einen Arzt über ein entsprechendes zentrales Servicecenter zur Verfügung zu stellen.

Am distalen Ende der Elektrodenleitung 405 kann in unmittelbarer Nähe des jeweils wahrnehmenden Elektrodenpols ein Wandler 480 angebracht sein, der die von den wahmehmenden Elektrodenpolen erfassten Signale (beispielsweise Potentialdifferenzen) derart wandelt, dass sie ungestört zur Auswerteeinheit übertragen werden können. Ein derartiger Wandler 480 ist als optionale Komponente in Figuren 5a und 5b eingezeichnet und über eine Signalleitung SL mit der Auswerteeinheit 450 verbunden.

Der Wandler 480 kann beispielsweise ein Transformator sein, der das von den wahrnehmenden Elektrodenpolen erfasste Signal hochtransformiert, um es über hochohmige Leitungen (Signalleitung SL) gegenüber hochfrequenten Wechselfeldern störungsfest zur Auswerteeinheit 450 zu leiten.

Alternativ kann der Wandler 480 auch ein piezoelektrischer Wandler sein, der die von den wahrnehmenden Elektrodenpolen erfassten Signale beispielsweise in mechanische, konkret akustische Ausgangssignale wandelt, die über eine geeignete Signalleitung zur Auswerteeinheit 450 geleitet werden kann. Die Auswerteeinheit 450 kann auch ihrerseits einen akustoelektrischen Wandler aufweisen, der akustische Signale zurück in elektrische Signale wandelt.

Gemäß einer weiteren Alternative kann der Wandler 480 ein Gleichrichter oder Demodulator sein, dessen Ausgangssignal ein Gleichsignal ist, so dass eventuelle hochfrequente Störungen dieses Gleichsignals von der Auswerteeinheit 450 leicht herausgefiltert werden können.

Gemäß einer weiteren Alternative kann der Wandler 480 ein elektrooptischer Wandler sein, beispielsweise eine Leuchtdiode (LED), die das von den wahrnehmenden Elektrodenpolen erfasste Signal in ein Lichtsignal wandelt, das über einen Lichtleiter als Signalleiter SL ungestört zur Auswerteeinheit 450 geleitet werden kann. Die Auswerteeinheit 450 besitzt dann einen komplementären Wandler, der die Lichtsignale wieder in elektrische Signale wandelt, beispielsweise eine Fotodiode.

Der Wandler zum Wandeln des akustischen Signals oder des Lichtsignals in ein elektrisches Signal kann auch am proximalen Ende der Elektrodenleitung 405 vorgesehen sein. Dies ist in Figur 5B durch den Wandler 490 angedeutet, der über eine elektrische Signalleitung SL' mit der Auswerteeinheit 450 verbunden ist.

## Patentansprüche

1. Temporär oder permanent implantierbares medizinisches Gerät (10) mit wenigstens einem langgestreckten elektrischen Funktionsleiter (ZL1, ZL2, ZL3, 421, 422) für die Übertragung von Therapiesignalen oder Diagnosesignalen oder beidem, und einem mit dem Funktionsleiter (ZL1, ZL2, ZL3, 421, 422) verbundenen Elektrodenpol (22, 24, 30, 32, 210, 220, 230, 310, 320), über den elektrischer Strom an im Benutzungsfall an umgebendes Körpergewebe abgegeben oder mit dem elektrische Potentiale in im Benutzungsfall umgebenden Gewebe abgefühlt werden können oder beides,
**dadurch gekennzeichnet, dass** das medizinische Gerät
eine Wahrnehmungseinrichtung (460), die mit einem felderzeugenden sowie einem potentialabfühlenden Elektrodenpol (22, 24, 30, 32, 210, 220, 230, 310, 320) verbunden und ausgebildet ist, im Falle einer durch hochfrequente, äußere Störfelder induzierten Stromabgabe über den felderzeugenden Elektrodenpol (22, 24, 30, 32, 210, 220, 230, 310, 320) generierter, das umliegende Gewebe erwärmender elektrischer Potentiale über den potentialabfühlenden Elektrodenpol (22, 24, 30, 32, 210, 220, 230, 310, 320) mit Bezug auf ein Bezugspotential zu erfassen und ein ein erfasstes elektrisches Potential repräsentierendes Ausgangssignal zu erzeugen,
sowie eine Steuereinrichtung (410) aufweist, die mit der Wahrnehmungseinrichtung (460) verbunden und ausgebildet ist, ein von der Wahrnehmungseinrichtung (460) erzeugte Ausgangssignal auszuwerten und das medizinische Gerät (10) in Abhängigkeit des von der Wahrnehmungseinrichtung (460) erfassten Potentials zu steuern.

2. Medizinisches Gerät nach Anspruch 1, **dadurch gekennzeichnet, dass** der potentialabfühlende Elektrodenpol (22, 24, 30, 32, 210, 220, 230, 310, 320) gleichzeitig der stromabgebende Elektrodenpol (22, 24, 30, 32, 210, 220, 230, 310, 320) ist.

3. Medizinisches Gerät nach Anspruch 1, **dadurch gekennzeichnet, dass** der potentialabfühlende Elektrodenpol (22, 24, 30, 32, 210, 220, 230, 310, 320) und der stromabgebende Elektrodenpol (22, 24, 30, 32, 210, 220, 230, 310, 320) separate Elektrodenpole sind.

4. Medizinisches Gerät nach einem der Ansprüche 1 bis 3, **dadurch gekennzeichnet, dass** der im Betriebsfall das Bezugspotential liefernde Anschluss ein dem felderzeugenden Elektrodenpol (22, 24, 30, 32, 210, 220, 230, 310, 320) zugeordneter Anschluss ist.

5. Medizinisches Gerät nach einem der Ansprüche 1 bis 3, **dadurch gekennzeichnet, dass** der im Betriebsfall das bezugspotential liefernde Anschluss ein dem wahrnehmenden Elektrodenpol (22, 24, 30, 32, 210, 220, 230, 310, 320) zugeordneter Anschluss ist.

6. Medizinisches Gerät nach einem der Ansprüche 1 bis 3 mit einem Gehäuse, **dadurch gekennzeichnet, dass** die Wahrnehmungseinrichtung mit dem Gehäuse (12, 400) verbunden ist, so dass dieses im Betriebsfall das Bezugspotential liefert.

7. Medizinisches Gerät nach einem der Ansprüche 1 bis 3, **dadurch gekennzeichnet, dass** in unmittelbarer Nähe des wahrnehmenden Elektrodenpols (22, 24, 30, 32, 210, 220, 230, 310, 320) oder der wahrnehmenden Elektrodenpole (22, 24, 30, 32, 210, 220, 230, 310, 320) ein Wandler (480) angeordnet ist, der das mit dem wahrnehmenden Elektrodenpol (22, 24, 30, 32, 210, 220, 230, 310, 320) erfasste Potential in ein störunempfindliches Signal umwandelt.

8. Medizinisches Gerät nach Anspruch 7, **dadurch gekennzeichnet, dass** der Wandler (480) ein Transformator ist, der ausgebildet ist, das erfasste Potential hochzutransformieren, um es über hochohmige Leitungen (SL) zur Auswerteeinheit (460) zu leiten

9. Medizinisches Gerät nach Anspruch 7, **dadurch gekennzeichnet, dass** der Wandler (480) ein elektroakustischer, insbesondere ein piezoelektrischer Wandler ist, der ausgebildet ist, das erfasste Potential in ein mechanisches, insbesondere akustisches Signal zu wandeln.

10. Medizinisches Gerät nach Anspruch 7, **dadurch gekennzeichnet, dass** der Wandler (480) ein Gleichrichter/Demodulator ist, dazu ausgebildet ist, das erfasste Potential in ein Gleichsignal zu wandeln.

11. Medizinisches Gerät nach Anspruch 7, **dadurch gekennzeichnet, dass** der Wandler (480) ein elektrooptischer Wandler, insbesondere eine Leuchtdiode ist, der dazu ausgebildet ist, dass erfasste Potential in ein Lichtsignal zu wandeln.

12. Medizinisches Gerät nach Anspruch 9 oder 11 in Form einer Elektrodenleitung (20) mit einem distalen und einem proximalen Ende, bei der der wahrnehmende Elektrodenpol (22, 24, 30, 32, 210, 220, 230, 310, 320) im Bereich des distalen Endes angeordnet ist, **dadurch gekennzeichnet, dass** am proximalen Ende der Elektrodenleitung (20) ein zweiter Wandler (480) angeordnet ist, der ausgebildet ist, das mechanische oder optische Signal ist ein elektrisches Signal zu wandeln.

13. Medizinisches Gerät nach einem der Ansprüche 1 bis 12, **dadurch gekennzeichnet, dass** die Wahrnehmungseinrichtung (460) eine Auswerteeinrichtung mit einem Schwellwertdetektor (440) und einer Auswerteeinheit (450) umfasst.

14. Medizinisches Gerät nach Anspruch 13, **dadurch gekennzeichnet, dass** die Auswerteeinrichtung (460) ausgebildet ist, Signale für vorgegebene Frequenzen oberhalb einer vorgegebenen Schwelle auszuwerten.

## Claims

1. A medical device (10) that can be implanted temporarily or permanently, comprising at least one elongate electric functional conductor (ZL1, ZL2, ZL3, 421, 422) for the transmission of therapeutic signals or diagnostic signals or both, and comprising an electrode pole (22, 24, 30, 32, 210, 220, 230, 310, 320), which is connected to the functional conductor (ZL1, ZL2, ZL3, 421, 422) and via which electric current can be delivered to surrounding bodily tissue during use or by means of which the electric potentials in surrounding tissue during use can be sensed or both,
**characterised in that** the medical device
comprises a sensing arrangement (460), which is connected to a field-generating and to a potential-sensing electrode pole (22, 24, 30, 32, 210, 220, 230, 310, 320) and which is designed, in the event of a current delivery induced by high-frequency external interference fields, to detect in relation to a reference potential, via the potential-sensing electrode pole (22, 24, 30, 32, 210, 220, 230, 310, 320), electric potentials which are generated via the field-generating electrode pole (22, 24, 30, 32, 210, 220, 230, 310, 320) and which heat the surrounding tissue and to generate an output signal representing a detected electric potential,
and comprises a control arrangement (410), which is connected to the sensing arrangement (460) and which is designed to evaluate an output signal generated by the sensing arrangement (460) and to control the medical device (10) in accordance with the potential detected by the sensing arrangement (460).

2. The medical device according to Claim 1, **characterised in that** the potential-sensing electrode pole (22, 24, 30, 32, 210, 220, 230, 310, 320) is simultaneously the current-delivering electrode pole (22, 24, 30, 32, 210, 220, 230, 310, 320).

3. The medical device according to Claim 1, **characterised in that** the potential-sensing electrode pole (22, 24, 30, 32, 210, 220, 230, 310, 320) and the current-delivering electrode pole (22, 24, 30, 32, 210, 220, 230, 310, 320) are separate electrode poles.

4. The medical device according to one of Claims 1 to 3, **characterised in that** the connection delivering the reference potential during operation is a connection associated with the field-generating electrode pole (22, 24, 30, 32, 210, 220, 230, 310, 320).

5. The medical device according to one of Claims 1 to 3, **characterised in that** the connection delivering the reference potential during operation is a connection associated with the sensing electrode pole (22, 24, 30, 32, 210, 220, 230, 310, 320).

6. The medical device according to one of Claims 1 to 3, comprising a housing, **characterised in that** the sensing arrangement is connected to the housing (12, 400) such that this delivers the reference potential during operation.

7. The medical device according to one of Claims 1 to 3, **characterised in that** a converter (480) is arranged in the immediate vicinity of the sensing electrode pole (22, 24, 30, 32, 210, 220, 230, 310, 320) or of the sensing electrode poles (22, 24, 30, 32, 210, 220, 230, 310, 320) and converts the potential detected by means of the sensing electrode pole (22, 24, 30, 32, 210, 220, 230, 310, 320) into a signal that is not susceptible to interference.

8. The medical device according to Claim 7, **characterised in that** the converter (480) is a transformer that is designed to step-up the detected potential in order to convey it via high-resistance lines (SL) to the evaluation unit (460).

9. The medical device according to Claim 7, **characterised in that** the converter (480) is an electroacoustic converter, in particular a piezoelectric converter, which is designed to convert the detected potential into a mechanical signal, in particular an acoustic signal.

10. The medical device according to Claim 7, **characterised in that** the converter (480) is a rectifier/demodulator designed to convert the detected potential into a DC signal.

11. The medical device according to Claim 7, **characterised in that** the converter (480) is an electro-optical converter, in particular a light-emitting diode, which is designed to convert the detected potential into a light signal.

12. The medical device according to Claim 9 or 11 in the form of an electrode line (20) having a distal and a proximal end, in which the sensing electrode pole (22, 24, 30, 32, 210, 220, 230, 310, 320) is arranged in the region of the distal end, **characterised in that** a second converter (480) is arranged at the proximal end of the electrode line (20) and is designed to convert the mechanical or optical signal into an electric signal.

13. The medical device according to one of Claims 1 to 12, **characterised in that** the sensing arrangement (460) comprises an evaluation arrangement having a threshold value detector (440) and an evaluation unit (450).

14. The medical device according to Claim 13, **characterised in that** the evaluation arrangement (460) is designed to evaluate signals for predefined frequencies above a predefined threshold.

## Revendications

1. Appareil médical implantable temporaire ou permanent (10) avec au moins un conducteur électrique fonctionnel allongé (ZL1, ZL2, ZL3, 421, 422) pour la transmission de signaux thérapeutiques ou de signaux de diagnostic ou des deux, et un pôle d'électrode (22, 24, 30, 32, 210, 220, 230, 310, 320) relié au conducteur fonctionnel (ZL1, ZL2, ZL3, 421, 422), par lequel un courant électrique est délivré à des tissus corporels environnants pendant l'utilisation ou par lequel des potentiels électriques peuvent être détectés dans les tissus environnants pendant l'utilisation, ou les deux,
**caractérisé en ce que** l'appareil médical
comporte un dispositif de perception (460) relié à un pôle d'électrode générateur de champ ainsi qu'à un pôle d'électrode détecteur de potentiel (22, 24, 30, 32, 210, 220, 230, 310, 320) et conçu pour détecter, dans le cas d'une délivrance de courant induite par des champs parasites extérieurs à haute fréquence, des potentiels électriques chauffant les tissus environnants, générés par le pôle d'électrode générateur de champ (22, 24, 30, 32, 210, 220, 230, 310, 320), par le biais du pôle d'électrode détecteur de potentiels (22, 24, 30, 32, 210, 220, 230, 310, 320), par rapport à un potentiel de référence, et pour produire un signal de sortie représentant un potentiel électrique détecté,
ainsi qu'un dispositif de commande (410) relié au dispositif de perception (460) et conçu pour évaluer un signal de sortie généré par le dispositif de perception (460) et pour commander l'appareil médical (10) en fonction du potentiel détecté par le dispositif de perception (460).

2. Appareil médical selon la revendication 1, **caractérisé en ce que** le pôle d'électrode détecteur de potentiels (22, 24, 30, 32, 210, 220, 230, 310, 320) constitue simultanément le pôle d'électrode générateur de champ (22, 24, 30, 32, 210,220,230,310,320).

3. Appareil médical selon la revendication 1, **caractérisé en ce que** le pôle d'électrode détecteur de potentiels (22, 24, 30, 32, 210, 220, 230, 310, 320) et le pôle d'électrode générateur de champ (22, 24, 30, 32, 210, 220, 230, 310, 320) sont des pôles d'électrode séparés.

4. Appareil médical selon l'une des revendications 1 à 3, **caractérisé en ce que** pendant l'utilisation, la borne fournissant le potentiel de référence est une borne attribuée au pôle d'électrode générateur de champ (22, 24, 30, 32, 210, 220, 230, 310, 320).

5. Appareil médical selon l'une des revendications 1 à 3, **caractérisé en ce que** pendant l'utilisation, la borne fournissant le potentiel de référence est une borne attribuée au pôle d'électrode de perception (22, 24, 30, 32, 210, 220, 230, 310, 320).

6. Appareil médical selon l'une des revendications 1 à 3, avec un logement, **caractérisé en ce que** le dispositif de perception est relié au logement (12, 400), de sorte que celui-ci fournit le potentiel de référence pendant l'utilisation.

7. Appareil médical selon l'une des revendications 1 à 3, **caractérisé en ce qu'**à proximité immédiate du pôle d'électrode de perception (22, 24, 30, 32, 210, 220, 230, 310, 320) ou des pôles d'électrode de perception (22, 24, 30, 32, 210, 220, 230, 310, 320) est agencé un convertisseur (480) convertissant le potentiel détecté par le pôle d'électrode de perception (22, 24, 30, 32, 210, 220, 230, 310, 320) en un signal insensible aux parasites.

8. Appareil médical selon la revendication 7, **caractérisé en ce que** le convertisseur (480) est un transformateur conçu pour augmenter la tension du potentiel détecté, pour le guider vers l'unité d'évaluation (460) par des lignes à haute impédance (SL).

9. Appareil médical selon la revendication 7, **caractérisé en ce que** le convertisseur (480) est un convertisseur électroacoustique, en particulier piézoélectrique, conçu pour convertir le potentiel détecté en un signal mécanique, en particulier acoustique.

10. Appareil médical selon la revendication 7, **caractérisé en ce que** le convertisseur (480) est un redresseur/démodulateur conçu pour convertir le potentiel détecté en un signal continu.

11. Appareil médical selon la revendication 7, **caractérisé en ce que** le convertisseur (480) est un convertisseur électro-optique, en particulier une diode électroluminescente conçue pour convertir le potentiel détecté en un signal lumineux.

12. Appareil médical selon la revendication 9 ou 11, sous la forme d'une ligne d'électrodes (20) avec une extrémité distale et une extrémité proximale, dans laquelle le pôle d'électrode de perception (22, 24, 30, 32, 210, 220, 230, 310, 320) est disposé dans la région de l'extrémité distale, **caractérisé en ce qu'**à l'extrémité proximale de la ligne d'électrodes (20) est agencé un deuxième convertisseur (480) conçu pour convertir le signal mécanique ou optique en un signal électrique.

13. Appareil médical selon l'une des revendications 1 à 12, **caractérisé en ce que** le dispositif de perception (460) comprend un dispositif d'évaluation avec un détecteur de valeur seuil (440) et une unité d'évaluation (450).

14. Appareil médical selon la revendication 13, **caractérisé en ce que** le dispositif d'évaluation (460) est conçu pour évaluer des signaux pour des fréquences prédéfinies au-delà d'un seuil prédéfini.
